# EUROPEAN PATENT SPECIFICATION

(11) **EP 0 501 679 B1**
(45) Date of publication and mention of the grant of the patent: **15.05.1996**
(21) Application number: 92301423.7
(22) Date of filing: 20.02.1992
(51) Int. Cl.: A61L 17/00

(54) **Preparation process for surgical filament**
Verfahren zur Herstellung eines chirurgischen Nähfadens
Procédé de préparation d'un filament chirurgical

(30) Priority: 27.02.1991 JP 32839/91
(43) Date of publication of application: 02.09.1992
(73) Proprietor: MITSUI TOATSU CHEMICALS, Inc., Chiyoda-Ku Tokyo 100 (JP)
(72) Inventor: Shinoda, Hosei, Kasugai-shi, Aichi-ken 487 (JP); Ohtaguro, Masami, Nagoya-shi, Aichi-ken 458 (JP); Iimuro, Shigeru, Nagoya-shi, Aichi-ken 458 (JP)
(74) Representative: Nicholls, Kathryn Margaret

(56) References cited:
- EP-A- 0 436 308
- FR-A- 2 297 051

## Description

The present invention relates to a process for preparing a surgical filament such as a surgical suture and a ligature having improved surface-slipping characteristics, for example, passing ability through the tissue and tie down property.

Various surgical filaments such as sutures and ligatures are used in surgery for the suture, fixation and ligation of tissues including the internal organs, skin, muscles, bones, joints and blood vessels.

These surgical filaments are sometimes used for sutures and ligatures in the form of a monofilament. However, these filaments are often used in the form of multifilament or as a braided or twisted structure.

Even in the case of a surgical filament composed of monofilaments having relatively good surface-slipping characteristics, untreated surfaces of the monofilament sometimes damages tissue by non-negligible friction between the filament and the tissue.

In order to prevent such problem or to slide a knot of the suture to a desired position, various coatings are applied to the surgical filaments.

For example, Japanese Patent Publication SHO 60-25974(1985) discloses a synthetic multi-filament suture covered with a mixture composed of a metal salt of higher fatty acid and a bioabsorbable polymer.

Japanese Laid-Open Patent SHO 61-76163(1986) also describes a surgical filament dry-coated with a metal salt of higher fatty acid.

The surgical filaments are, in use, that is, in the course of surgery, usually exposed to body fluid or passed through a wet tissue from once to several times before making a knot.

When a conventionally known coating material, for example, calcium stearate is applied to the surface of the bioabsorbable suture, a certain extent of lubricating effect can be observed in the dry state. On the other hand, the surface-slipping characteristics are sometimes impaired in the wet state and thus it becomes difficult to slide down a knot of the suture to the desired position. Additionally, there is a problem that passing ability through the tissue is unsatisfactory.

The present inventors disclosed in the earlier Japanese Laid-Open Patent HEI 3-236852(1991) (corresponding to the earlier European patent application EP-A-0436308) that a surgical filament having a surface coated with at least one kind of N-long chain monoacylated basic amino acid which has an aliphatic acyl group of from 6 to 22 carbon atoms or with a composition containing the basic amino acid has surface-slipping characteristics such as tie down property and passing ability through the tissue which are maintained substantially constant in both wet and dry states and additionally has a high safety for organism.

According to the disclosure, the application of at least one kind of N-long chain monoacylated basic amino acid or a composition containing the amino acid to the surface of the surgical filament is carried out by a method for directly attaching the powder of the amino acid to the surface of the surgical filament without solvent, or a method for passing the filament through a suspension of the amino acid in a solvent and successively removing the solvent to yield a dry coated filament, or a method for adhering the amino acid to the surface of the filament in the form of a composition containing the amino acid and a bioabsorbable and thermoplastic or solvent-soluble polymer. The amount of the N-long chain monoacylated basic amino acid is from 0.1 to 20 parts by weight per 100 parts by weight of the filament.

As a result of further investigation, however, the present inventors have found that these methods are not always satisfactory because of the following reason. Generally marketed N-long chain monoacylated basic amino acid is a white flaky crystal having an average particle size of 20 to 30 µm and a maximum particle size of about 100 µm. When the N-long chain monoacylated basic amino acid is attached to the surface of the surgical filament, large particles of the amino acid are adhered intact and sometimes lead to various problems.

For example, when powder containing a large particle size of at least one kind of the N-long chain monoacylated basic amino acid or the amino acid composition is directly attached to the surface of the filament without using a solvent, or when the powder of the composition containing the amino acid is dispersed in a molten, thermoplastic, bioabsorbable polymer and adhered to the surface of the filament, the amount required to coat the surface is at least 20 % by weight based on the weight of the filament in order to obtain satisfactory surface-slipping property. A coating amount exceeding 10 % by weight or application of the powder containing a large particle size of the N-long chain monoacylated basic amino acid leads to unfavorable reduction of commodity value because the surface of the filament looks like a wheat flour coated surface and becomes pale white.

Further, the N-long chain monoacylated basic amino acid having a large particle size has a small area of adhesion per unit weight and is hence liable to cause shortage in adhesive force.

Consequently, when the surgical filament coated with the N-long chain monoacylated basic amino acid is packaged, transported, stored or used as a suture or a ligature, the powder of N-long chain monoacylated basic amino acid falls off and contaminates the surroundings or the surface-slipping characteristics of the filament such as tie down property and passing ability through the tissue are substantially decreased.

When a knot is made on a surgical filament or tied down to the suture area in a practical operation of surgical suture, the powder of N-long chain monoacylated basic amino acid falls off by friction and substantially decreases the surface-slipping characteristics. Further, released powder unfavorably scatters over the diseased part of a human body and it is required to wash and remove the powder.

Embodiments of the present invention desirably provide a process for the preparation of a surgical filament which has good appearance and which retaines the coating agent during packaging, transportation, storage and use as a suture or a ligature.

Embodiments of the present invention also desirably provide a process for preparing a surgical filament which can maintain surface-slipping characteristics substantially constant in both wet and dry states though coated with a small amount of the coating agent and which has a high safety for the organism.

As a result of an intensive investigation, the present inventors have found that by applying to the surface of a surgical filament a coating of N-long chain monoacylated basic amino acid having a specific particle size the problems of the prior art filaments may be ameliorated. Thus the present invention has been completed.

That is, the aspect of the invention is a preparation process for a surgical filament comprising coating the surface of the surgical filament by using a coating material selected from the group consisting of (1) the powder of at least one kind of N-long chain monoacylated basic amino acid having an aliphatic acyl group of from 6 to 22 carbon atoms wherein 80 % by volume or more of particles has a size of 20 µm or less, (2) a suspension of said powder, and (3) a composition containing said powder.

The present invention is more preferably a preparation process for a surgical suture comprising coating a surgical filament with said N-long chain monoacylated basic amino acid which has a volume percentage of 80 % or more on particles having a size of 20 µm or less and additionally has an average particle size of 7 µm or less.

The present invention provides a process for preparating the surgical filament which can maintain the surface-slipping characteristics such as tie down property and passing ability through the tissue substantially constant in both wet and dry states and has a high safety for the organism.

That is, the surgical filament prepared by the process of the invention has good appearance and retains the coating agent during packaging, transportation, storage and use as a suture or a ligature. Further, good slipping characteristics can be provided for the surgical filament with a smaller amount of coating. These effects can be remarkably observed in particular when the surgical filament has a braided structure.

Embodiments of the invention are described below by way of example only with reference to the accompanying drawings of which:
Figure 1 is a drawing illustrating the procedure of tying which is carried out in a tie down test;
Figure 2 is a drawing schematically illustrating the equipment used for the tie down test.

1. Section of a bar
2. Filament
2a and 2b. Both ends of the filament
3. Knot
4. Sponge
5a and 5b. Chucks of the tensile tester
6. Load cell
7. Recorder
The N-long chain monoacylated basic amino acid which has an aliphatic acyl group of from 6 to 22 carbon atoms and is preferably used for the present invention is a compound represented by the formula (I) : 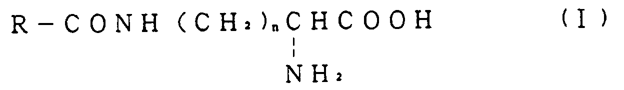
wherein R is a long chain alkyl group having from 5 to 21 carbon atoms and n is an integer of from 1 to 4.

The N-long chain monoacylated basic amino acid of the invention may be coated singly on the filament or may be used as a component of the coating composition composed of several components. In the composition containing at least one kind of the N-long chain monoacylated basic amino acid, bioabsorbable polymers are preferably used as other components. The bioabsorbable polymers include, for example, polyglycolic acid, polylactic acid and a copolymer of glycolic acid and lactic acid which are polymers of glycolide and/or lactide.

When the term 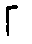 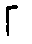 coating 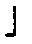 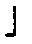 is used in the invention, coating is not necessarily required to cover the whole surface of the filament. Mottled covering or spotted adhesion of the coating agent may also be partially found on the filament surface.

Embodiments of the invention will hereinafter be illustrated in detail.

The N-long chain monoacylated basic amino acid used in the invention has a structure that an amino group in a basic amino acid having a plurality of amino group is acylated with a long chain amino acid.

In such N-long chain monoacylated basic amino acid, the basic amino acid includes, for example, α,β-diaminopropionic acid, α,γ-diaminobutyric acid, ornithine and lysine. Lysine exhibits a particularly preferred coating effect, that is, surface-slipping characteristics.

The long chain acyl group preferably has from 6 to 22 carbon atoms and includes, for example, acyl groups derived from caproic acid, enanthic acid, caprylic acid, pelargonic acid, capric acid, lauric acid, myristic acid, palmitic acid, stearic acid, icosanoic acid, docosanic acid, oleic acid, behenic acid, isostearic acid, coconut oil fatty acid and beef tallow fatty acid. Preferred acids are saturated fatty acids such as lauric acid, myristic acid, palmitic acid and stearic acid. Lauric acid is particularly preferred.

N-Acylated basic amino acids having an acyl group of less than 6 carbon atoms are too hydrophilic and cannot exhibit good coating effects. The acyl group having more than 22 carbon atoms also cannot provide good effects.

Consequently, the N-long chain monoacylated basic amino acid for use in the invention is preferred. A particularly preferred compound is N-ε-lauroyl-L-lysine which has the formula (II) : 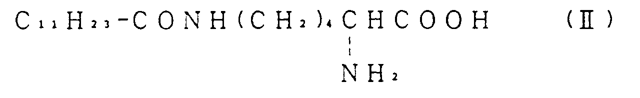
The N-long chain monoacylated basic amino acid is prepared, for example, by Shotten Bauman's reaction in which long chain carboxylic acid chloride is added dropwise to an aqueous alkali solution of basic amino acid or by a method disclosed in Japanese Patent Publication SHO 51-128610(1976), in which the carboxylic acid salt of the basic amino acid is dehydrated by heating.

However, no particular restriction is imposed on the method of synthetics of the N-long chain monoacylated basic amino acid and the amino acid prepared by other methods can also be used without any troubles.

The N-long chain monoacylated basic amino acid typically has the form of white flake crystals having a melting point of from 200 to 250°C, having excellent lubricating properties, being insoluble or slightly soluble in water and common organic solvents such as chloroform and benzene, and additionally which do not swell and can be maintained in the form of a stable powder. Consequently, frictional resistance on the filament surface is effectively decreased by coating the N-long chain monoacylated basic amino acid on the filament.

Since the N-long chain monoacylated basic amino acid has good water repellency, the surgical filament surface coated with said substance sheds water effectively when the filament is exposed to body fluids or passes through wet tissue. As a result, the filament can exhibit slipping characteristics substantially equal to those in the dry state.

Further, the N-long chain monoacylated basic amino acid is extremely safe for organisms as understood from its chemical structure. When the long chain monoacyl group is a fatty acid in particular, neither acute nor subacute toxicity is found and any test results on skin irritation, mucous membrane irritation and mutagenicity are negative. Hence, it is reasonable to use said substance for the coating agent.

The surgical filament of the invention may be bioabsorbable or non-bioabsorbable and also may have a monofilament structure or a ultra fine multifilament structure. A surgical filament having a braided structure of a bioabsorbable multifilament in particular exhibits the advantageous effects of the coating of the present invention.

That is, the N-long chain monoacylated basic amino acid which used in the present invention preferably has specific particle size and is relatively small particles. Hence the particle of the N-long chain monoacylated basic amino acid penetrates in good order into the gaps among multifilament and can provide uniform coating over the whole surface of the filament.

Consequently, good slipping characteristics can be exhibited even with a small amount of coating, and appearance of the surgical filament is not impaired.

Further, the particle of N-long chain monoacylated basic amino acid seldom falls off from the surface of coated filaments in the packaging, transportation, storage and use such as suture.

The material of the bioabsorbable surgical filament includes, for example, conventionally known natural substances such as catgut, collagen and chitin, and synthetic substances such as glycolide polymers (polyglycolic acid), lactide polymers (polylactic acid), glycolide-lactide copolymers, glycolide-caprolactone copolymers, polyhydroxybutyric acid, poly-p-dioxanone and trimethylene carbonate polymers.

Glycolide polymers, lactide polymers and glycolide-lactide copolymers are preferred among these polymers in view of the effect of coating on improving the surface-slipping characteristics and filament properties such as high strength and good hydrolyzability.

The characteristic of the present invention is to use the N-long chain monoacylated basic amino acid having a specific particle size.

That is, the N-long chain monoacylated basic amino acid has a volume percentage of 80 % or more on particles having a size of 20 µm or less, preferably has a volume percentage of 80 % or more on particles having a size of 20 µm or less and additionally has an average particle size of 7 µm or less.

The N-long chain monoacylated basic amino acid having such particle size distribution can be prepared by grinding and/or sieving the N-long chain monoacylated basic amino acid obtained by the above process.

No particular restriction is imposed on the method for grinding the N-long chain monoacylated basic amino acid. Conventionally used mechanical-impact grinders such as a vibrating ball-mill and a hammer bell-mill or other grinders such as jet grinders which use high speed gas flow can be preferably used. The latter is preferred in particular.

No particular limitation is placed on the method for classifying the powder or fine particles which are ground as above. Preferred methods are classification using a conventional multicyclone or a combination of several standard sieves specified in JIS Z-8801.

When a particle size distribution to meet the object of the invention, that is, a volume percentage of 80 % or more on particles having a size of 20 µm or less cannot be obtained by grinding and classification in any of the above methods, grinding and classification are repeated to obtain particles having a suitable size distribution for the object.

In the present invention, the N-long chain monoacylated basic amino acid having the above particle size distribution (hereinafter referred to as N-long chain monoacylated basic amino acid) is applied to the surface of the surgical filament intact or in the form of a composition with other materials.

The present invention is characterized by the particle size distribution of the coating material used. No particular restriction is imposed upon the coating method applied. Exemplary methods which are preferably used include :
(1) a method for directly coating the powder or fine particles of the N-long chain monoacylated basic amino acid on the surface of the surgical filament,
(2) a method for dispersing the powder or fine particles of the N-long chain monoacylated basic amino acid in water or an organic solvent and coating the resulting suspension on the filament, and
(3) a method for dispersing the powder or fine particles of the N-long chain monoacylated basic amino acid in a hot melt or solution of a bioabsorbable polymer and coating the resulting suspension on the filament.

Alternatively, it is possible to dissolve the powder or fine particles of the N-long chain monoacylated basic amino acid or a composition containing the amino acid in a suitable organic solvent and coating the resulting solution on the filament. The particles of the amino acid used are relatively small in size and can be dissolved in the organic solvent with relative ease. However, the effect of solubility is not so remarkable and the solution method is not so suitable for the invention.

In the above method (1), the powder or fine particles of the N-long chain monoacylated basic amino acid is directly coated on the surface of the surgical filament at or in the vicinity of the room temperature with a finger or a brush.

Other methods which can be exemplified include a so-called spray coating method, i.e., a method for spraying the powder of the amino acid on the surface of the filament by using stream of the air, nitrogen or other inert gases which are blown under the pressure of 0.5 to 5 atmospheres at or in the vicinity of the room temperature.

As an example of the above method (2), the powder or fine particles of the N-long chain monoacylated basic amino acid are dispersed at or in the vicinity of the room temperature in water or an organic solvent such as chloroform, dioxane, toluene, xylene, methanol and ethanol, and the filament is coated by passing through the resulting suspension with stirring, followed by evaporating the solvent. Other methods include, for example, a spray coating method, i.e., a method for spraying the resulting suspension by using stream of the air, nitrogen or other inert gases which are blown under the pressure of 0.5 to 5 atmospheres.

The concentration of the suspension is preferably in the range of 2 to 100 parts by weight of the coating materials per 100 parts by weight of the suspending medium such as water. A concentration less than 2 parts by weight leads to an insufficient amount of the coating material adhered. On the other hand, a concentration exceeding 100 parts by weight causes unfavorable precipitation of the coating materials and thus it becomes difficult to obtain a good suspension.

In the preparation of the above suspension, a small amount of a surface active agent or emulsifier can also be added in order to stabilize the suspension.

The evaporation temperature of the solvent used for dispersing the coating materials, that is, the drying temperature of the filament depends upon the volatility and boiling point of the solvent used. For example, in the case of using toluene as a suspending medium, a preferred temperature is in the range of 40 to 120 °C in an inert gas such as the air or nitrogen.

Batch and continuous dryers can be used. It is important to maintain inside and outlet temperatures of the dryers above the dew-point of the suspending medium such as toluene.

As an embodiment of the above method (3), the powder or fine particles of the N-long chain monoacylated basic amino acid is dispersed in a hot melt of a biocompatible polymer or a bioabsorbable polymer, and the surgical filament is coated by passing through the resulting dispersion and cooled. Alternatively, the biocompatible polymer or the bioabsorbable polymer is dissolved in an organic solvent such as toluene, xylene, chloroform and dioxane, the powder or fine particles of the N-long chain monoacylated basic amino acid are dispersed in the resulting solution, the surgical filament is passed through the resulting suspension or spray coated the resulting suspension under pressure of 0.5 to 5 atmospheres, and thus-coated filament is dried.

The polymers used in the above method preferably have biocompatibility, bioabsorbability and thermoplastic property as the surgical filament.

The polymers which can be used include, for example, glycolide polymers, lactide polymers, glycolide-lactide copolymers, polycaprolactone, polyoxyalkylene, polytetrafluoroethylene and silicone resin. Particularly preferred polymers are bioabsorbable polymers such as glycolide polymers, lactide polymers and glycolide-lactide copolymers.

When a hot melt of glycolide-lactide copolymers or polycaprolactone is used, the polymers is heat-melted at 100 to 200°C, successively the N-long chain monoacylated basic amino acid is added, and the surgical filaments is passed through the molten mixture thus obtained to carry out coating. Then the coated filament is cooled in water or in the air. The preferred proportion of the N-long chain monoacylated basic amino acid in the composition is from 30 to 200 parts by weight per 100 parts by weight of the above polymer. When the proportion is outside of the above range, adhesion of the composition to the filament surface becomes unfavorably soft and weak.

In another method, the above polymer is dissolved in a solvent such as toluene, xylene, chloroform and dioxane in the range of room temperature to 100°C and successively the N-long chain monoacylated basic amino acid is added to form a suspension.

The surgical filament is passed through the suspension or sprayed the suspension under the pressure of 0.5 to 5 atmospheres to carry out coating and successively dried.

The coating temperature is preferably from room temperature to 50°C. Higher temperature is unfavorable because solvent evaporation causes time dependent variation of concentration. Immersion of the filament in the suspension for 2 seconds to one minute can provide satisfactory coating.

The suspension is prepared by dissolving from 0.01 to 10 parts by weight of the above polymer in 100 parts by weight of the organic solvent, adding from 0.1 to 20 parts by weight of the N-long chain monoacylated basic amino acid, and maintaining at room temperature to 50°C under stirring. Stirring is continued from the preparation of the dispersion to the completion of coating in order to eliminate variation of concentration of the N-long chain monoacylated basic amino acid. After completion of the coating, the filament is dried in air or inert gas such as nitrogen for 1 to 24 hours in the range of from room temperature to 120°C to remove the organic solvent. Drying under reduced pressure is more effective.

Other conventionally known lubricants or coating additives can be used in combination with the N-long chain monoacylated basic amino acid in coating the filament of the invention. Exemplary lubricants and coating additives include calcium stearate, magnesium stearate, barium stearate, aluminum stearate, zinc stearate, calcium palmitate, magnesium palmitate, barium palmitate, aluminum palmitate, zinc palmitate, calcium oleate, magnesium oleate, barium oleate, aluminum oleate, zinc oleate and other metal salts of fatty acids having at least 6 carbon atoms; and esters of sucrose with fatty acids having at least 6 carbon atoms.

In these simultaneous uses, the preferred ratio of the components is from 20 to 100 % by weight of the N-long chain monoacylated basic amino acid to from 0 to 80 % by weight of the fatty acid metal salt.
When the amount of the N-long chain monoacylated basic amino acid is less than 20 % by weight or less, improvement of surface-slipping characteristics is unsatisfactory in the wet state.

Combined use of the metal salts of fatty acids having at least 6 carbon atoms or esters of sucrose with fatty acids having at least 6 carbon atoms can be carried out in the above methods (1) to (3). However, combined use can be more preferably carried out, for example, by adding the above fatty acid metal salt in an organic solvent such as xylene, refluxing for 30 to 60 minutes with stirring at the boiling point of the solvent, cooling the mixture to 30 to 80 °C to obtain a gelated dispersion of the fatty acid metal salt in the organic solvent, successively dispersing the powder of the N-long chain monoacylated basic amino acid with stirring, passing the surgical filament through the resulting suspension, and drying the thus-coated filament.

The amount of the N-long chain monoacylated basic amino acid to be coated on the surface of the filament in the invention varies depending upon the structure of the filament, for example, the number of filaments and whether the filaments is braided or twisted, and is suitably selected in the range of from 0.1 to 10 % by weight for the weight of the filament.

When the amount is less than 0.1 % by weight, development of a good coating effect is difficult to realize. An amount exceeding 10 % by weight unfavorably leads to high cost. A more preferred amount is in the range of from 0.5 to 5.0 % by weight.

The present invention will hereinafter be illustrated further in detail by way of examples. Physical properties in the examples were measured by the following methods.

### Tie down test

### (1) Subjective method

As to the surgical filament provided by the invention, evaluation of slipping characteristics, particularly tie down property of a knot, can be carried out with extreme ease in a subjective method.

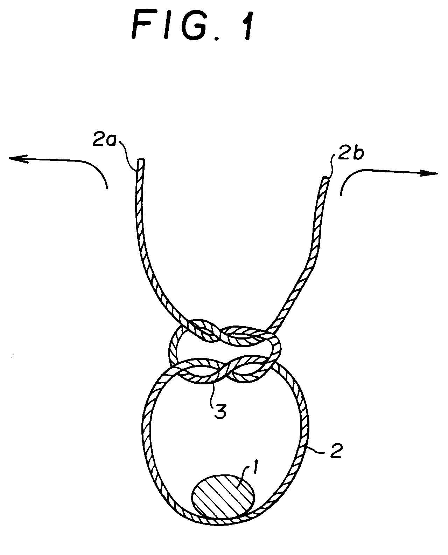

As illustrated in Figure 1, a filament 2 which was passed under a bar 1 was firmly tied at the upper parts and then the resulting knot 3 was pulled down by drawing both ends 2a and 2b of the filament in the directions of the arrows, respectively. The slipping property could be readily evaluated by the ease of pull down.

### (2) Objective method

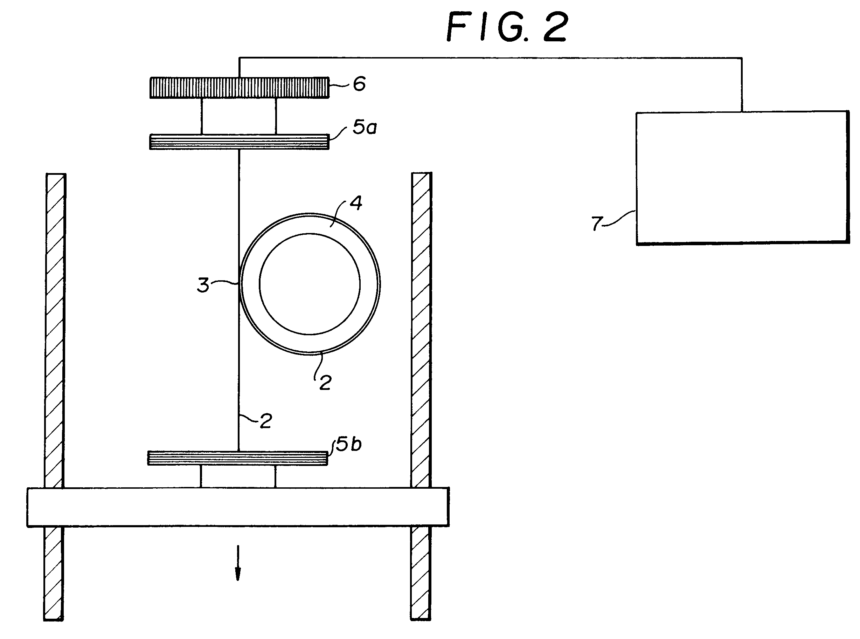

As illustrated in Figure 2, a filament 2 to be tested was turned once around a cylindrical sponge 4 having an inside diameter of 40 mm and an outside diameter of 50 mm and a knot 3 was made as illustrated in Figure 1.
Both ends of the filament 2 were respectively fixed to chucks 5a and 5b of a tensile testing machine and drawn at a rate of 100 mm/min. The knot did not slide in some of the filaments 2 and the filament 2 was broken. When the knot could slide, the knot was subjected to slide a distance of 30 mm. The stress detected by a load cell 6 varied depending upon the slide distance of the filament 2. Hence, the initial 10 mm and final 10 mm were omitted from the total slide distance of 30 mm, and maximum and minimum stresses were recorded on the intermediate 10 mm.

### Grinding of N-lauroyl lysine and measurement of its particle size distribution

Marketed N-lauroyl lysine was ground using a single track jet mill. Model STD-4 (Trade mark of Seishin Kigyo Co.) and recovered with a Teflon bag filter.

N-lauroyl lysine having two kinds of particle size distribution was obtained by grinding and individually referred to as sample 1 and sample 2 of the coating material. Unground N-lauroyl lysine was referred to as sample 3.

Two or three drops of marketed liquid neutral detergent were added to 400 mℓ of pure water to prepare an aqueous solution containing a very small amount of surface active agent. Each 0.5 g of the above samples was added to the aqueous solution and dispersed for 3 minutes by supersonic waves.

The particle size distribution of each sample in the dispersion was measured with a light irradiation type particle size measuring instrument, Model SK-LASER MICRON SISER 7000 (Trade mark of Seishin Kigyo Co.). The particle size wherein accumulated volume percentage of particle size becomes 50 vol. % in each sample was defined as an average particle size. Table 1 illustrates the particle size distribution and average particle size of each sample.

### Examples 1-4 and Comparative Examples 1-4

All surgical filaments used had a multifilament braided structure. Their sizes are illustrated in Table 2 based on the specification of United States Pharmacopeia XIX (hereinafter referred to as USP).

The powder of sample 1, sample 2 or sample 3 of the coating material having various particle sizes which are illustrated in Table 1 was directly adhered to a surgical filament with a human finger and excess coating material was wiped off by rubbing the filament with a dry cloth. According to the number of whiping cycles by the cloth, filaments having various amounts of the coating agents adhered were obtained.
The weight of the filaments thus obtained was measured. The adhered amount of the coating material was calculated from the difference in weight between the coated and originals filaments, and indicated by percentage to the weight of filament.

Slipping characteristics of coated filaments in the dry state were evaluated by the objective method.

Coated filaments were immersed in distilled water at 37°C for a minute and slipping characteristics in the wet state were evaluated by the objective method as above.

The filaments coated with sample 1 and sample 2, respectively, had good slipping characteristics in spite of a small amount of coating. No falling off of the coating material was observed. Results are illustrated in Table 2.

As clearly seen in Table 2, Example 1 and Comparative Example 2, Example 2 and Comparative Example 3, and Example 4 and Comparative Example 4 are individually almost equal in the amount of the coating material adhered. In any case, the examples had better slipping characteristics of filament surface than the Comparative Examples. The effect of specifying the particle size of the coating material could be observed. Comparative Example 4 had great absolute values of surface slipping characteristics and additionally had a large difference between maximum and minimum values.

In all Examples, no fall off the coating material was observed in the tie down procedures and appearance of the coated filaments was good.

### Examples 5-7 and Comparative Examples 5-7

Chloroform solutions of resin were prepared by adding 1 g of the resin illustrated in Table 3 to a prescribed amount of chloroform and successively stirring at room temperature for 30 minutes.

Suspensions of the coating material were successively prepared by adding the prescribed amounts of sample 1 or sample 3 of the coating material having various particle sizes illustrated in Table 1 to the above-obtained solution with stirring.

The same filaments as used in Example 1 were immersed in the above suspensions for about 10 seconds to coat various resins and samples. Successively, the filaments were dried in the air at 50°C for an hour to obtain coated filaments.

The adhered amount of the coating material to the filament is indicated by the total amount of the above resin and sample 1 or sample 3.

The tie down property of the filament at the wet and dry states were evaluated by the subjective method.
Examples 5, 6 and 7 had better surface tie down property than Comparative Examples 5, 6 and 7, respectively, in spite of less amount of coating. All examples which used sample 1 of the coating material caused no fall off of the coating material in the tie down procedures. Further, the coated filament had good appearance. Results are illustrated in Table 3.

### Example 8

A xylene solution of poly-D,L-lactic acid having an average molecular weight of 5000 was prepared by adding 2 g of the resin to 94 g of xylene and stirring for 30 minutes at room temperature.

Successively, a suspension of the coating material was prepared by adding 4 g of sample 1 illustrated in Table 1 to the xylene solution with stirring.

The resin and sample 1 were coated on the same kind of a filament as used in Example 1 by immersing the filament in the suspension with stirring. The coated filament was dried by a hot air stream at 50 °C for an hour.

The adhered amount of the coating material was 2.3 % by weight. Surface slipping characteristic of the coated filament was evaluated by the subjective method as carried out in Example 1. As a result, evaluation was grade 4. The coated filament had good appearance. No fall off of the coating material was observed in the tie down test.

### Example 9

A suspension of the coating material illustrated in Table 1 was prepared by adding 80 g of sample 1 to 1000 mℓ of xylene and stirring vigorously.

The suspension was sprayed on the same kind of filament as used in Example 1 at the pressure of 3 kg/cm² with a marketed manual atomizer having a content of 1000 mℓ and a nozzle diameter of 2 mm. The coated filament was dried in the air at 100 °C for 30 minutes.

The procedures of coating and drying were repeated three times to obtain a filament having 6.5 % by weight of coated material for the weight of the filament.

The tie down property of the coated filament was evaluated by the subjective method as carried out in Example 1.

As a result, surface slipping characteristic was grade 4 in both wet and dry states. Thus slipping characteristic was good. No fall off of the coating material was observed in the procedures for making a knot and tying down. The coated filament had good appearance.

### Example 10

A gel dispersion was prepared by adding 40 g of calcium stearate to 1000 mℓ of xylene, refluxing at 150 °C for 30 minutes with stirring, and cooling to 50 °C

A suspension of the coating material illustrated in Table 1 was prepared by adding 40 g of sample 1 to the gel dispersion obtained above and stirring vigorously.

The same coating procedures as described in Example 8 were carried out except that the suspension obtained above was used. A filament having 5.8 % by weight of coated material was obtained and evaluated by the same method as described in Example 8.

As a result, surface slipping characteristic was grade 4 in both wet and dry states. Thus slipping characteristic was good. No fall off of the coating material was observed in the procedures for making a knot and tying down. The coated filament had good appearance.

**Table 1**

| Particle size | Accumulated volume distribution of particle size (Vol. %) | | |
|---|---|---|---|
| (µm) | Sample 1 | Sample 2 | Sample 3 |
| 0.1 | 0.0 | 0.0 | 0.0 |
| 0.2 | 0.0 | 0.0 | 0.0 |
| 0.4 | 0.9 | 0.3 | 0.1 |
| 0.6 | 3.1 | 0.7 | 0.2 |
| 0.8 | 7.5 | 1.2 | 0.3 |
| 1.0 | 14.9 | 1.9 | 0.4 |
| 1.5 | 26.9 | 3.5 | 0.7 |
| 2.0 | 47.3 | 8.5 | 1.8 |
| 3.0 | 71.4 | 16.9 | 4.4 |
| 4.0 | 88.2 | 29.4 | 6.5 |
| 6.0 | 97.9 | 46.8 | 8.5 |
| 8.0 | 97.9 | 62.5 | 11.8 |
| 12.0 | 97.9 | 79.9 | 17.4 |
| 16.0 | 97.9 | 89.1 | 28.7 |
| 24.0 | 97.9 | 96.8 | 54.0 |
| 32.0 | 100.0 | 100.0 | 72.2 |
| 48.0 | 100.0 | 100.0 | 91.9 |
| 64.0 | 100.0 | 100.0 | 97.0 |
| 96.0 | 100.0 | 100.0 | 99.4 |
| 128.0 | 100.0 | 100.0 | 100.0 |
| 192.0 | 100.0 | 100.0 | 100.0 |
| Average particle size (µm) | 2.1 | 6.4 | 22.7 |

**Table 2**

| | Example | | | | Comparative Example | | | |
|---|---|---|---|---|---|---|---|---|
| | 1 | 2 | 3 | 4 | 1 | 2 | 3 | 4 |
| Kind of filament* | 2-0 | 2-0 | 3-0 | 2 | 2-0 | 2-0 | 2-0 | 2 |
| Sample No. | 1 | 1 | 2 | 1 | 3 | 3 | 3 | 3 |
| Adhered amount (wt %) | 3.7 | 1.9 | 2.9 | 0.6 | 7.1 | 3.8 | 2.0 | 0.5 |

| Slipping characteristics | | | | | | | | |
|---|---|---|---|---|---|---|---|---|
| Dry(Kgf) | 0.1∼ 0.2 | 0.1∼ 0.2 | 0.1∼ 0.2 | 0.2∼ 0.4 | 0.1∼ 0.3 | 0.2∼ 0.3 | 0.3∼ 0.4 | 0.4∼ 0.9 |
| Wet(Kgf) | 0.1∼ 0.2 | 0.1∼ 0.3 | 0.2∼ 0.3 | 0.3∼ 0.5 | 0.2∼ 0.3 | 0.3∼ 0.4 | 0.4∼ 0.6 | 0.5∼ 1.1 |
| Appearance | good | good | good | good | white | white | pale white | good |
| Fall off in tie down | no | no | no | no | violent | found | found | no |

| | | | | | | | | |
|---|---|---|---|---|---|---|---|---|
| Note: * Polyglycolide filament Size is indicated according to USPXIX specification. | | | | | | | | |

**Table 3**

| | Example | | | Comparative Example | | |
|---|---|---|---|---|---|---|
| | 5 | 6 | 7 | 5 | 6 | 7 |
| Kind of filament* | 2 - 0 | 2 - 0 | 2 - 0 | 2 - 0 | 2 - 0 | 2 - 0 |
| Chloroform (mℓ) | 40 | 40 | 70 | 40 | 40 | 70 |
| Coating resin(g)** | 1.0 | 1.0 | 1.0 | 1.0 | 1.0 | 1.0 |
| Sample No. | 1 | 1 | 1 | 3 | 3 | 3 |
| N-Lauroyl lysine(g) | 1.0 | 2.0 | 4.0 | 1.0 | 2.0 | 4.0 |
| Adhered amount (wt%) | 3.2 | 4.3 | 1.9 | 3.6 | 5.2 | 2.0 |

| Tie down | | | | | | |
|---|---|---|---|---|---|---|
| Dry | 4 | 4 | 3 | 3 | 3 | 2 |
| Wet | 3 | 4 | 3 | 2 | 3 | 1 |
| Appearance | good | good | good | pale white | white | pale white |
| Fall off in tie down | no | no | no | found | found | no |

| | | | | | | |
|---|---|---|---|---|---|---|
| Note: * Polyglycolide. Size is illustrated according to USP XIX specification. | | | | | | |
| ** Poly-L-lactide | | | | | | |
| *** Tie down property was classified into five grades according to the following standards for evaluation. 0 : Knot does not slide at all or filament is broken. 1 : Knot moves only slightly. Considerable force is required for moving the knot. 2 : Knot moves with intermittent hang up. 3 : Knot can be slided down with ease. 4 : Knot slides down very smoothly. | | | | | | |

## Claims

1. A process for preparing a coated surgical filament comprising coating the surface of a surgical filament with a coating material selected from the group consisting of (1) powder of at least one of N-long chain monoacylated basic amino acid having an aliphatic acyl group of from 6 to 22 carbon atoms wherein 80% by volume or more of particles has a size of 20 µm or less (2) a suspension of said powder; and (3) a composition containing said powder.

2. The process of claim 1 wherein the amount of coating is from 0.1 to 10% by weight of the surgical filament.

3. The process of claim 1 or claim 2 wherein the suspension is obtained by suspending from 2 to 100 parts by weight of the powder of the N-long chain monoacylated basic amino acid in 100 parts by weight of a suspending medium selected from water, chloroform, dioxane, toluene, xylene, methanol and ethanol.

4. The process of claim 1 or claim 2 wherein the suspension is obtained by dissolving from 0.01 to 10 parts by weight of a bioabsorbable polymer selected from glycolide polymer, lactide polymer and glycolide-lactide copolymer to form a solution and suspending from 0.1 to 20 parts by weight of the powder of the N-long chain monoacylated basic amino acid in the solution.

5. The process of claim 1 or claim 2 wherein the composition contains from 100 parts by weight of hot melt of a bioabsorbable polymer selected from glycolide polymer, lactide polymer and glycolide-lactide copolymer and from 30 to 200 parts by weight of the powder of the N-long chain monoacylated basic amino acid.

6. The process of claim 1 or claim 2 wherein the composition contains from 20 to 100% by weight of the powder of the N-long chain monoacylated basic amino acid and from 0 to 80% by weight of a metal salt of fatty acid having 6 or more of carbon atoms.

7. The process of any one of the preceding claims wherein the surgical filament is a bioabsorbable polymer.

8. The process of claim 7 wherein the bioabsorbable polymer is glycolide polymer, lactide polymer or glycolide-lactide copolymer.

9. The process of any one of the preceding claims wherein the surgical filament has a multifilament braided structure.

10. The process of any one of the preceding claims wherein the powder of N-long chain monoacylated basic amino acid having an acyl group of from 6 to 22 carbon atoms has a particle size of 20µm or less and an average particle size of 7 µm or less.

11. The process of any one of the preceding claims wherein the N-long chain monoacylated basic amino acid having an acyl group of from 6 to 22 carbon atoms is a compound represented by the formula (I): 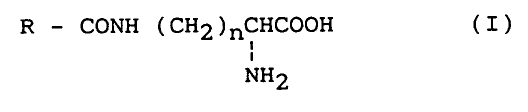 wherein R is an alkyl group having from 5 to 21 carbon atoms and n is an integer of from 1 to 4.

12. The process of claim 11 wherein R is an undecyl group and n is 4 in the formula (I).

13. A surgical filament as produced by the process of any one of claims 1 to 12.

## Patentansprüche

1. Verfahren zur Herstellung eines beschichteten chirurgischen Nähfadens, umfassend das Beschichten der Oberfläche eines chirurgischen Nähfadens mit einem Beschichtungsmaterial, ausgewählt aus der Gruppe bestehend aus (1) einem Pulver aus zumindest einer am N langkettig monoacylierten, basischen Aminosäure mit einer aliphatischen Acylgruppe mit 6 - 22 C-Atomen, worin 80 Vol.-% oder mehr der Teilchen eine Größe von 20 µm oder weniger aufweisen, (2) einer Suspension dieses Pulvers und (3) einer das Pulver enthaltenden Zusammensetzung.

2. Verfahren nach Anspruch 1, worin die Menge der Beschichtung 0,1 - 10 Gew.-% des chirurgischen Nähfadens beträgt.

3. Verfahren nach Anspruch 1 oder 2, worin die Suspension durch Suspendieren von 2 - 100 Gew.-Teilen des Pulvers der am N langkettig monoacylierten, basischen Aminosäure in 100 Gew.-Teilen eines aus Wasser, Chloroform, Dioxan, Toluol, Xylol, Methanol und Ethanol ausgewählten Suspensionsmediums gebildet wird.

4. Verfahren nach Anspruch 1 oder 2, worin die Suspension duch Auflösen von 0,01 - 10 Gew.-Teilen eines aus Glykolidpolymer, Lactidpolymer und Glykolid-Lactidcopolymer ausgewählten, bioabsorbierbaren Polymers zur Bildung einer Lösung und durch Suspendieren von 0,1 - 20 Gew.-Teilen des Pulvers der am N langkettig monoacylierten, basischen Aminosäure in der Lösung erhalten wird.

5. Verfahren nach Anspruch 1 oder 2, worin die Zusammensetzung 100 Gew.-Teile einer heißen Schmelze eines aus Glykolidpolymer, Lactidpolymer und Glykolid-Lactid-Copolymer ausgewählten, bioabsorbierbaren Polymers sowie 30 - 200 Gew.-Teile des Pulvers der am N langkettig monoacylierten, basischen Aminosäure enthält.

6. Verfahren nach Anspruch 1 oder 2, worin die Zusammensetzung 20 - 100 Gew.-% des Pulvers der am N langkettig monoacylierten, basischen Aminosäure und 0 - 80 Gew.-% eines Metallsalzes einer Fettsäure mit 6 oder mehr C-Atomen enthält.

7. Verfahren nach einem der vorhergehenden Ansprüche, worin der chirurgische Nähfaden ein bioabsorbierbares Polymer ist.

8. Verfahren nach Anspruch 7, worin das bioabsorbierbare Polymer ein Glykolidpolymer, Lactidpolymer oder Glykolid-Lactidcopolymer ist.

9. Verfahren nach einem der vorhergehenden Ansprüche, worin der chirurgische Nähfaden eine Multifilament-Flechtstruktur aufweist.

10. Verfahren nach einem der vorhergehenden Ansprüche, worin das Pulver der am N langkettig monoacylierten, basischen Aminosäure mit einer Acylgruppe mit 6 - 22 C-Atomen eine Teilchengröße von 20 µm oder weniger und eine durchschnittliche Teilchengröße von 7 µm oder weniger aufweist.

11. Verfahren nach einem der vorhergehenden Ansprüche, worin die am N langkettig monoacylierte, basische Aminosäure mit einer Acylgruppe mit 6 - 22 C-Atomen eine durch die Formel (I): 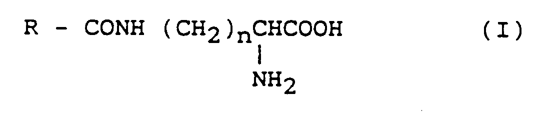 dargestellte Verbindung ist, worin R eine Alkylgrupe mit 5 - 21 C-Atomen und n eine ganze Zahl von 1 - 4 ist.

12. Verfahren nach Anspruch 11, worin in Formel (I) R eine Undecylgruppe und n = 4 ist.

13. Chirurgischer Nähfaden, hergestellt nach dem Verfahren nach einem der Ansprüche 1 - 12.

## Revendications

1. Procédé pour préparer un filament chirurgical revêtu consistant à revêtir la surface d'un filament chirurgical avec un matériau de revêtement choisi dans le groupe comprenant (1) une poudre d'au moins un amino-acide basique monoacylé à longue chaîne-N possédant un groupe acyle aliphatique de 6 à 22 atomes de carbone dans laquelle 80% en volume ou plus de particules ont une dimension de 20 µm ou moins, (2) une suspension de ladite poudre, et (3) une composition contenant ladite poudre.

2. Procédé selon la revendication 1, dans lequel la quantité de revêtement représente 0,1 à 10% en poids du filament chirurgical.

3. Procédé selon la revendication 1 ou la revendication 2, dans lequel la suspension est obtenue par mise en suspension de 2 à 100 parties en poids de la poudre de l'amino-acide basique monoacylé à longue chaîne-N dans 100 parties en poids d'un milieu de suspension choisi parmi l'eau, le chloroforme, le dioxane, le toluène, le xylène, le méthanol et l'éthanol.

4. Procédé selon la revendication 1 ou la revendication 2, dans lequel la suspension est obtenue par dissolution de 0,01 à 10 parties en poids d'un polymère bioabsorbable choisi parmi un polymère de glycolide, un polymère de lactide et un copolymère de glycolide-lactide pour former une solution et par mise en suspension de 0,1 à 20 parties en poids de la poudre de l'amino-acide basique monoacylé à longue chaîne-N dans la solution.

5. Procédé selon la revendication 1 ou 2, dans lequel la composition contient à partir de 100 parties en poids d'une masse fondue à chaud d'un polymère bioabsorbable choisi parmi un polymère de glycolide, un polymère de lactide et un copolymère de glycolide-lactide et de 30 à 200 parties en poids de la poudre de l'amino-acide basique monoacylé à longue chaîne-N.

6. Procédé selon la revendication 1 ou la revendication 2, dans lequel la composition contient de 20 à 100% en poids de la poudre de l'amino-acide basique monoacylé à longue chaîne-N et de 0 à 80% en poids d'un sel métallique d'acide gras ayant 6 ou davantage d'atomes de carbone.

7. Procédé selon l'une des revendications précédentes, dans lequel le filament chirurgical est un polymère bioabsorbable.

8. Procédé selon la revendication 7, dans lequel le polymère bioabsorbable est un polymère de glycolide, un polymère de lactide ou un copolymère de glycolide-lactide.

9. Procédé selon l'une quelconque des revendications précédentes, dans lequel le filament chirurgical a une structure multifilamentaire tressée.

10. Procédé selon l'une quelconque des revendications précédentes, dans lequel la poudre de l'amino-acide basique monoacylé à longue chaîne-M ayant un groupe acyle de 6 à 22 atomes de carbone possède une dimension de particules de 20 µm ou moins et une dimension moyenne de particules de 7 µm ou moins.

11. Procédé selon l'une quelconque des revendications précédentes, dans lequel l'amino-acide basique monoacylé à longue chaîne-N ayant un groupe acyle de 6 à 22 atomes de carbone est un composé représenté par la formule (I) 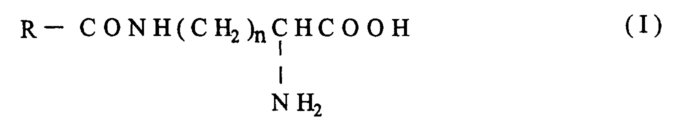 où R est un groupe alkyle ayant de 5 à 21 atomes de carbone et n est un nombre entier compris entre 1 et 4.

12. Procédé selon la revendication 11, dans lequel R est un groupe undécyle et n est égal à 4 dans la formule (I).

13. Filament chirurgical tel que produit par le procédé de l'une quelconque des revendications 1 à 12.
